# EUROPÄISCHE PATENTANMELDUNG

(11) **EP 2 085 487 A1**
(43) Veröffentlichungstag der Anmeldung: **05.08.2009**
(21) Anmeldenummer: 09157992.0
(22) Anmeldetag: 25.10.2002
(51) Int. Cl.: C12Q 1/68

(54) **Verfahren zum Nachweis von Cytosin-Methylierungen in immobilisierten DNA Proben**

(30) Priorität: 26.10.2001 DE 10154317
(62) Teilanmeldung aus: 02802274.7
(71) Anmelder: Epigenomics AG, 10178 Berlin (DE)
(72) Erfinder: Berlin, Kurt, 14532, Stahnsdorf (DE); Ballhause, Matthias, 13187, Berlin (DE); Gütig, David, 12159, Berlin (DE)

(57) **Zusammenfassung**

Beschrieben wird ein Verfahren zur Analyse von Cytosin-Methylierungsmustern in genomischen DNA-Proben. Im ersten Verfahrensschritt wird die genomische DNA aus Zellen oder anderen begleitenden Materialien isoliert und im wesentlichen irreversibel an eine Oberfläche gebunden. Danach wird die an die Oberfläche gebundene DNA bevorzugt mit einem Bisulfit derart behandelt, dass Cytosin in eine vom Basenpaarungsverhalten in der DNA-Duplex her unterschiedliche Base umgewandelt wird, während 5-Methylcytosin unverändert bleibt. Anschliessend werden die verwendeten Reagenzien in einem Waschschritt entfernt. Zuletzt werden ausgewählte Abschnitte der immobilisierten DNA in einer Polymerasereaktion amplifiziert und die Amplifikate werden hinsichtlich ihrer Sequenz untersucht.

## Beschreibung

### Gebiet der Erfindung

Die vorliegende Erfindung betrifft ein Verfahren zum Nachweis von Cytosin-Methylierung in DNA-Proben.

Die nach den methodischen Entwicklungen der letzten Jahre in der Molekularbiologie gut studierten Beobachtungsebenen sind die Gene selbst, die Übersetzung dieser Gene in RNA und die daraus entstehenden Proteine. Wann im Laufe der Entwicklung eines Individuums welches Gen angeschaltet wird und wie Aktivieren und Inhibieren bestimmter Gene in bestimmten Zellen und Geweben gesteuert wird, ist mit Ausmaß und Charakter der Methylierung der Gene bzw. des Genoms korrelierbar. Insofern äußern sich pathogene Zustände in einem veränderten Methylierungsmuster einzelner Gene oder des Genoms.

5-Methylcytosin ist die häufigste kovalent modifizierte Base in der DNA eukaryotischer Zellen. Sie spielt beispielsweise eine Rolle in der Regulation der Transkription, beim genetischen Imprinting und in der Tumorgenese. Die Identifizierung von 5-Methylcytosin als Bestandteil genetischer Information ist daher von erheblichem Interesse. 5-Methylcytosin-Positionen können jedoch nicht durch Sequenzierung identifiziert werden, da 5-Methylcytosin das gleiche Basenpaarungsverhalten aufweist wie Cytosin. Darüber hinaus geht bei einer PCR-Amplifikation die epigenetische Information, welche die 5-Methylcytosine tragen, vollständig verloren.

Eine relativ neue und die mittlerweile am häufigsten angewandte Methode zur Untersuchung von DNA auf 5-Methylcytosin beruht auf der spezifischen Reaktion von Bisulfit mit Cytosin, das nach anschließender alkalischer Hydrolyse in Uracil umgewandelt wird, welches in seinem Basenpaarungsverhalten dem Thymidin entspricht. 5-Methylcytosin wird dagegen unter diesen Bedingungen nicht modifiziert. Damit wird die ursprüngliche DNA so umgewandelt, dass Methylcytosin, welches ursprünglich durch sein Hybridisierungsverhalten vom Cytosin nicht unterschieden werden kann, jetzt durch "normale" molekularbiologische Techniken als einzig verbliebenes Cytosin beispielsweise durch Amplifikation und Hybridisierung oder Sequenzierung nachgewiesen werden kann. Alle diese Techniken beruhen auf Basenpaarung, welche jetzt voll ausgenutzt wird. Der Stand der Technik, was die Empfindlichkeit betrifft, wird durch ein Verfahren definiert, welches die zu untersuchende DNA in einer Agarose-Matrix einschließt, dadurch die Diffusion und Renaturierung der DNA (Bisulfit reagiert nur an einzelsträngiger DNA) verhindert und alle Fällung- und Reinigungsschritte durch schnelle Dialyse ersetzt (Olek A, Oswald J, Walter J. A modified and improved method for bisulphate based cytosine methylation analysis. Nucleic Acids Res. 1996 DEC 15;24(24):5064-6). Mit dieser Methode können einzelne Zellen untersucht werden, was das Potential der Methode veranschaulicht. Allerdings werden bisher nur einzelne Regionen bis etwa 3000 Basenpaare Länge untersucht, eine globale Untersuchung von Zellen auf Tausenden von möglichen Methylierungsanalysen ist nicht möglich. Allerdings kann auch dieses Verfahren keine sehr kleinen Fragmente aus geringen Probenmengen zuverlässig analysieren. Diese gehen trotz Diffusionsschutz durch die Matrix verloren.

Eine Übersicht über die weiteren bekannten Möglichkeiten, 5-Methylcytosine nachzuweisen, kann aus dem folgenden Übersichtsartikel entnommen werden:Rein T, DePamphilis ML, Zorbas H. Identifying 5-methylcytosine and related modifications in DNA genomes. Nucleic Acids Res. 1998 May 15;26(10):2255-64.

Die Bisulfit-Technik wird bisher bis auf wenige Ausnahmen (z. B. Zeschnigk M, Lich C, Buiting K, Dörfler W, Horsthemke B. A single-tube PCR test for the diagnosis of Angelman and Prader-Willi syndrome based on allelic methylation differences at the SNRPN locus. Eur J Hum Genet. 1997 Mar-Apr;5(2):94-8) nur in der Forschung angewendet. Immer aber werden kurze, spezifische Stücke eines bekannten Gens nach einer Bisulfit-Behandlung amplifiziert und entweder komplett sequenziert (Olek A, Walter J. The pre-implantation ontogeny of the H19 methylation imprint. Nat Genet. 1997 Nov.;17(3):275-6) oder einzelne Cytosin-Positionen durch eine "Primer-Extension-Reaktion" (Gonzalgo ML, Jones PA. Rapid quantitation of methylation differences at specific sites using methylation-sensitive single nucleotide primer extension (Ms-SNuPE). Nucleic Acids Res. 1997 Jun. 15;25(12):2529-31, WO-Patent 9500669) oder einen Enzymschnitt (Xiong Z, Laird PW. COBRA: a sensitive and quantitative DNA methylation assay. Nucleic Acids Res. 1997 Jun. 15;25(12):2532-4) nachgewiesen. Zudem ist auch der Nachweis durch Hybridisierung beschrieben worden (Olek et al., WO 99 28498).

Harnstoff verbessert die Effizienz der Bisulfit-Behandlung vor der Sequenzierung von 5-Methylcytosin in genomischer DNA (Paulin R, Grigg GW, Davey MW, Piper AA. Urea improves efficiency of bisulphate-mediated sequencing of 5'-methylcytosine in genomic DNA. Nucleic Acids Res. 1998 Nov. 1;26(21):5009-10).

Weitere Publikationen, die sich mit der Anwendung der Bisulfit-Technik zum Methylierungsnachweis bei einzelnen Genen befassen, sind:
Grigg G, Clark S. sequencing 5-methylcytosine residues in genomic DNA. Bioassays. 1994 Jun.;16(6):431-6, 431; Zeschnigk M, Schmitz B, Dittrich B, Buiting K, Horsthemke B, Dörfler W. Imprinted segments in the human genome: different DNA methylation patterns in the Prader-Willi/Angelman syndrome region as determined by the genomic sequencing method. Hum Mol Genet. 1997 Mar;6(3):387-95; Feil R, Charlton J, Bird AP, Walter J, Reik W. Methylation analysis on individual chromosomes: improved protocol fort bisulphate genomic sequencing. Nucleic Acids Res. 1994 Feb. 25;22(4):695-6; Martin V, Ribieras S, Song-Wang X, Rio MC, Dante R. Genomic sequencing indicates a correlation between DNA hypomethylation in the 5' region of the pS2 gene andin its expression in human breast cancer cell lines. Gene. 1995 May 19;157(1-2):261-4; WO 97/46705, WO 95/15373 und WO 95/45560.

Ein weiteres bekanntes Verfahren ist die sogenannte methylierungssensitive PCR (Herman JG, Graff JR, Myohanen S, Nelkin BD, Baylin SB. (1996), Methylation-specific PCR: a novel PCR assay for methylation status of CpG islands. Proc Natl Acad Sci U S A. Sep 3;93(18):9821-6). Für dieses Verfahren werden Primer verwendet, die entweder nur an eine Sequenz hybridisieren, die durch die Bisulfit-Behandlung einer an der betreffenden Position unmethylierten DNA entsteht, oder aber umgekehrt Primer, welche nur an eine Nukleinsäure bindet, die durch die Bisulfit-Behandlung einer an der betreffenden Position unmethylierten DNA entsteht. Mit diesen Primer können demnach Amplifikate erzeugt werden, deren Detektion wiederum Hinweise auf das Vorliegen einer methylierten oder unmethylierten Position in der Probe liefern, an welche die Primer binden.

Ein neueres Verfahren ist auch der Nachweis von Cytosin-Methylierung mittels einer Taqman PCR, das als Methyl-Light bekannt geworden ist (WO 00/70090). Mit diesem Verfahren ist es möglich, den Methylierungsstatus einzelner oder weniger Positionen direkt im Verlauf der PCR nachzuweisen, so dass sich eine nachfolgende Analyse der Produkte erübrigt.

Eine Übersicht über den Stand der Technik in der Oligomer Array Herstellung lässt sich aus einer im Januar 1999 erschienenen Sonderausgabe von Nature Genetics (Nature Genetics Supplement, Volume 21, January 1999), der dort zitierten Literatur und dem US-Patent 5994065 über Methoden zur Herstellung von festen Trägern für Zielmoleküle wie Oligonucleotide bei vermindertem nichtspezifischen Hintergrundsignal entnehmen.

Für die Abtastung eines immobilisierten DNA-Arrays sind vielfach fluoresziert markierte Sonden verwendet worden. Besonders geeignet für Fluoreszenzmarkierungen ist das einfache Anbringen von Cy3 und Cy5 Farbstoffen am 5'-OH der jeweiligen Sonde. Die Detektion der Fluoreszenz der hybridisierten Sonden erfolgt beispielsweise über ein Konfokalmikroskop. Die Farbstoffe Cy3 und Cy5 sind, neben vielen anderen, kommerziell erhältlich.

Matrix-assistierte Laser Desorptions/Ionisations-Massenspektro-metrie (MALDI-TOF) ist eine sehr leistungsfähige Entwicklung für die Analyse von Biomolekülen (Karas M, Hillenkamp F. Laser desorption ionization of proteins with molecular masses exceeding 10,000 daltons. Anal Chem. 1988 Oct. 15;60(20):2299-301). Ein Analyt wird in eine lichtabsorbierende Matrix eingebettet. Durch einen kurzen Laserpuls wird die Matrix verdampft und das Analytmolekül so unfragmentiert in die Gasphase befördert. Durch Stöße mit Matrixmolekülen wird die Ionisation des Analyten erreicht. Eine angelegte Spannung beschleunigt die Ionen in ein feldfreies Flugrohr. Auf Grund ihrer verschiedenen Massen werden Ionen unterschiedlich stark beschleunigt. Kleinere Ionen erreichen den Detektor früher als größere.

MALDI-TOF Spektroskopie eignet sich ausgezeichnet zur Analyse von Peptiden und Proteinen. Die Analyse von Nukleinsäuren ist etwas schwieriger (Gut, I. G. und Beck, S. (1995), DNA and Matrix Assisted Laser Desorption Ionization Mass Spectrometry. Molecular Biology: Current Innovations and Future Trends 1: 147-157.) Für Nukleinsäuren ist die Empfindlichkeit etwa 100 mal schlechter als für Peptide und nimmt mit zunehmender Fragmentgröße überproportional ab. Für Nukleinsäuren, die ein vielfach negativ geladenes Rückgrat haben, ist der Ionisationsprozess durch die Matrix wesentlich ineffizienter. In der MALDI-TOF Spektroskopie spielt die Wahl der Matrix eine eminent wichtige Rolle. Für die Desorption von Peptiden sind einige sehr leistungsfähige Matrices gefunden worden, die eine sehr feine Kristallisation ergeben. Für DNA gibt es zwar mittlerweile einige ansprechende Matrices, jedoch wurde dadurch der Empfindlichkeitsunterschied nicht verringert. Der Empfindlichkeitsunterschied kann verringert werden, indem die DNA chemisch so modifiziert wird, dass sie einem Peptid ähnlicher wird. Phosphorothioatnukleinsäuren, bei denen die gewöhnlichen Phosphate des Rückgrats durch Thiophosphate substituiert sind, lassen sich durch einfache Alkylierungschemie in eine ladungsneutrale DNA umwandeln (Gut, I. G. und Beck, S. (1995), A procedure for selective DNA alkylation and detection by mass spectrometry. Nucleic Acids Res. 23: 1367-1373). Die Kopplung eines "charge tags" an diese modifizierte DNA resultiert in der Steigerung der Empfindlichkeit um den gleichen Betrag, wie er für Peptide gefunden wird. Ein weiterer Vorteil von "charge tagging" ist die erhöhte Stabilität der Analyse gegen Verunreinigungen, die den Nachweis unmodifizierter Substrate stark erschweren.

Genomische DNA wird durch Standardmethoden aus DNA von Zell-, Gewebe- oder sonstigen Versuchsproben gewonnen. Diese Standardmethodik findet sich in Referenzen wie Fritsch und Maniatis, Molecular Cloning: A Laboratory Manual, 1989.

Nach der Erfindung der PCR sind in den folgenden Jahren zahlreiche Varianten bekannt geworden, die diese Technik zur Amplifikation der DNA verfeinern. Insbesondere ist hier die Multiplexierung der PCR (Multiplex-PCR) zu erwähnen, wobei man mehr als 2 spezifische Primer einsetzt und dabei in einem Reaktionsgefäß eine Vielzahl von verschiedenen, spezifischen Amplifikationen erzeugen kann. Besonders interessant ist auch die sogenannte Nested PCR, welche unter anderem zum Nachweis besonders geringer DNA Mengen verwendet wird. Diese Art der PCR besteht aus zwei aufeinanderfolgenden Amplifikationen, wobei die Primer der zweiten Amplifikation innerhalb des ersten Amplifikates liegen und nicht mit den Primern der ersten Amplifikation identisch sind. Dadurch wird eine besondere Spezifität erreicht, da die Primer der zweiten Amplifikation nur dann funktionieren, wenn in der ersten Amplifikation das beabsichtigte Fragment erzeugt wurde. Dagegen ist die Vermehrung etwaiger Nebenprodukte der ersten Amplifikation in der zweiten so gut wie ausgeschlossen.

Die vorliegenden Verfahren zur Methylierungsanalyse, welche eine Bisulfitreaktion beinhalten, haben durchweg den Nachteil, dass die Reaktionslösung nicht unmittelbar für eine nachfolgenden Polymerasekettenreaktion eingesetzt werden kann, da der hohe Salzgehalt der Bisulfitreaktion sich störend auswirkt. Es müssen also in der Praxis mehrere Aufreinigungs- und/oder Waschschritte durchgeführt werden, was insbesondere bei kleinen DNA-Probenmengen zu schlechter Reproduzierbarkeit der Protokolle, umständlicher Handhabung und geringer Empfindlichkeit der Methode beiträgt. Zudem muss die DNA erst, wie auch für andere molekularbiologische Assays, isoliert werden, bevor sie in der Bisulfitreaktion eingesetzt werden kann.

Aufgabe der vorliegenden Erfindung ist es daher, die Nachteile des Standes der Technik zu überwinden.

Die Aufgabe wird durch ein Verfahren zur Analyse von Cytosin-Methylierungsmustern in genomischen DNA-Proben gelöst, wobei man die folgenden Verfahrensschritte ausführt:
a) die genomische DNA wird aus Zellen oder anderen begleitenden Materialien isoliert und im wesentlichen irreversibel an eine Oberfläche gebunden,
b) die an die Oberfläche gebundene DNA wird, bevorzugt mit einem Bisulfit (=Disulfit, Hydrogensulfit) derart behandelt, dass Cytosin in eine vom Basenpaarungsverhalten in der DNA-Duplex her unterschiedliche Base umgewandelt wird, während 5-Methylcytosin unverändert bleibt,
c) die in Schritt b) verwendeten Reagenzien werden in einem Waschschritt entfernt,
d) ausgewählte Abschnitte der immobilisierten DNA werden in einer Polymerasereaktion amplifiziert,
e) die Amplifikate werden hinsichtlich ihrer Sequenz untersucht.
   Vorteilhaft ist es, dass man folgende zusätzliche Schritte ausführt:
f) die Reagenzien und Produkte der Polymerasereaktion werden in einem Waschschritt entfernt,
g) weitere ausgewählte Abschnitte, welche von denen in Schritt d) verschieden sind, der immobilisierten DNA werden in einer Polymerasereaktion amplifiziert,
h) die Amplifikate werden hinsichtlich ihrer Sequenz untersucht.

Es ist erfindungsgemäß weiterhin besonders vorteilhaft, dass man die Schritte f)-g) mehrmals wiederholt, wobei in jeder Amplifikation nach Schritt g) andere Abschnitte amplifiziert werden als in einer der vorangegangenen Amplifikationen.

Erfindungsgemäß bevorzugt ist es, dass die Bindung der DNA an die Oberfläche kovalent ist.

Besonders vorteilhaft ist es dabei, dass man die DNA unmittelbar in dem Immobilisierungsschritt auch isoliert.

Erfindungsgemäß bevorzugt ist es, dass die Isolierung der DNA aus Vollblut oder Serum erfolgt. Vorteilhaft ist es erfindungsgemäß auch, dass die Isolierung der DNA aus lysiertem Gewebe erfolgt.

Erfindungsgemäß bevorzugt ist es dabei, dass man die Lysis mittels Proteinase K durchführt.

Erfindungsgemäß bevorzugt ist es insbesondere, dass die Immobilisierung in den Gefäßen einer Mikrotiterplatte mit 96 Gefäßen oder 384 Gefäßen erfolgt, wobei in den Gefäßen unterschiedliche DNA-Proben immobilisiert werden.

Besonders vorteilhaft ist es erfindungsgemäß auch, dass die Immobilisierung in PCR-Reaktionsgefäßen erfolgt, wobei in den Gefäßen unterschiedliche DNA-Proben immobilisiert werden. Bevorzugt ist es erfindungsgemäß, dass die Immobilisierung der DNA an einem Metalloxid, bevorzugt Aluminiumoxid, erfolgt.

Vorteilhaft ist das erfindungsgemäße Verfahren auch, wenn die Immobilisierung an einem hydrophoben Material erfolgt und die Bindung nur unter den gewählten Pufferbedingungen im wesentlichen irreversibel ist.

Es ist erfindungsgemäß auch bevorzugt, dass ein Amplifikationsschritt mit mehreren Primerpaaren als Multiplex-PCR ausgeführt wird.

Bevorzugt ist es erfindungsgemäß dabei, dass alle oder ein großer Teil der Amplifikate einer immobilisierten DNA Probe gepoolt werden und so gemeinsam einer weiteren Analyse zugeführt werden. Ein großer Teil sind etwa 50% und mehr der Amplifikate. Es können aber auch bis zu 75 % und mehr sein.

Bevorzugt ist beim erfindungsgemäßen Verfahren auch, dass es sich bei dieser weiteren Analyse um die Hybridisierung an einen Oligonukleotidarray oder PNA (Peptide Nucleic Acids)-Array handelt.

Bevorzugt ist es erfindungsgemäß auch, dass die Analyse während der Amplifikation durch eine Realtime PCR Methode erfolgt.

Erfindungsgemäß vorteilhaft ist es auch, dass die Analyse nach der Amplifikation im gleichen Reaktionsgefäß durch die Aufnahme einer Schmelzkurve erfolgt.

Erfindungsgemäß besonders bevorzugt ist es, dass die Analyse durch allelspezifische Hybridisierung von Oligonukleotiden oder PNAs (Peptide Nucleic Acids) an die zu untersuchenden Positionen in den Amplifikaten erfolgt.

Erfindungsgemäß bevorzugt ist ferner, dass die Analyse durch Hybridisierung von Oligonukleotidprimern und einer nachfolgenden Primerextensionsreaktion oder einer Sequenzierreaktion erfolgt.

Gegenstand der vorliegenden Erfindung ist auch die Verwendung des erfindungsgemäßen Verfahrens zur Diagnose und/oder Prognose nachteiliger Ereignisse für Patienten oder Individuen, wobei diese nachteiligen Ereignisse mindestens einer der folgenden Kategorien angehören: unerwünschte Arzneimittelwirkungen; Krebserkrankungen; CNS-Fehlfunktionen, Schäden oder Krankheit; Aggressionssymptome oder Verhaltensstörungen; klinische, psychologische und soziale Konsequenzen von Gehirnschädigungen; psychotische Störungen und Persönlichkeitsstörungen; Demenz und/oder assoziierte Syndrome; kardiovaskuläre Krankheit, Fehlfunktion und Schädigung; Fehlfunktion, Schädigung oder Krankheit des gastrointestinalen Traktes; Fehlfunktion, Schädigung oder Krankheit des Atmungssystems; Verletzung, Entzündung, Infektion, Immunität und/oder Rekonvaleszenz; Fehlfunktion, Schädigung oder Krankheit des Körpers als Abweichung im Entwicklungsprozess; Fehlfunktion, Schädigung oder Krankheit der Haut, der Muskeln, des Bindegewebes oder der Knochen; endokrine und metabolische Fehlfunktion, Schädigung oder Krankheit; Kopfschmerzen oder sexuelle Fehlfunktion.

Bevorzugt ist erfindungsgemäß dabei, die Verwendung des erfindungsgemäßen Verfahrens zur Unterscheidung von Zelltypen oder Geweben oder zur Untersuchung der Zelldifferenzierung.

Gegenstand der vorliegenden Erfindung ist ferner ein Kit, bestehend aus einem Reagenz zur Behandlung der DNA gemäss Schritt b, mindestens zwei Primeroligonukleotiden zur Herstellung der Amplifikate, einer Festphase zur Immobilisierung der Proben-DNA, sowie optional weiteren Lösungen und einer Anleitung zur Durchführung eines Assays nach einem erfindungsgemäßen Verfahren.

Die der vorliegenden Erfindung zugrundeliegende Lösung des Problems besteht darin, dass DNA, die im Rahmen ihrer ohnehin angestrebten Isolierung aus beispielsweise Vollblut, Serum oder Gewebe ohnehin an eine Festphase gebunden wird, direkt auf dieser Festphase nachfolgend auch einer Bisulfitreaktion unterworfen wird. Nach der in diesem Falle sehr einfachen, durch Nachwaschen mit Wasser oder einem geeigneten Puffer zu erzielenden Abtrennung der Bisulfit-Reaktionsmischung kann die immobilisierte DNA unmittelbar auch direkt für die Amplifikation eingesetzt werden. Alternativ kann sie in dieser immobilisierten Form gelagert werden und erst bei Bedarf für eine Amplifikation verwendet werden. Da die immobilisierte DNA bei der Amplifikation nicht wesentlich verändert wird, ist es auch möglich, mit der immobilisierten DNA als Templat mehrere nachfolgende Amplifikationen durchzuführen, nachdem die Reaktionskomponenten der jeweils vorangegangenen Amplifikation durch Waschschritte entfernt wurden.

Insgesamt stellt die vorliegenden Erfindung also ein Verfahren zur Verfügung welches hinsichtlich der Durchführung beliebiger, auf Bisulfitbehandlung zurückgreifender Methylierungsassays eine erhebliche Vereinfachung darstellt. Die Proben-DNA muss nur noch direkt an eine Festphase gebunden werden, die Bisulfitbehandlung an dieser Festphase durchgeführt und nachfolgend unter Verwendung immer noch derselben Festphase eine Polymerasereaktion durchgeführt werden. Dies erlaubt es auch, stabile Assays ausgehend von sehr geringen DNA Mengen, etwa aus Serum, durchzuführen.

Sinnvollerweise ist die Festphase eine modifizierte Oberfläche eines Gefäßes, in welchem dann auch die PCR-Reaktion durchgeführt wird und vorteilhafterweise ein handelsübliches PCR Gefäß, welches auch als 8er Streifen oder als Teil einer Mikrotiterplatte vorliegen kann. Wesentliche Aufgabe der vorliegenden Erfindung war es also unter anderem, Oberflächen bereitzustellen, welche zum einen die DNA möglichst irreversibel binden können, zum anderen aber auch unter den in der Bisulfitbehandlung vorliegenden Bedingungen hinreichend stabil bleiben und die DNA weiterhin gebunden halten.

Es wurden zwei Oberflächen identifiziert, die diesen Zweck erfüllen. Zum einen handelt es sich um Aluminiumoxid, zum anderen um C18-Alkylketten, welche in Verbindung mit geeigneten Kationen wie Triethylammoniumionen eine feste Anbindung der DNA erlauben. C18-Alkylketten können durch dem Fachmann bekannte Silanisierungsverfahren mittels einem Octadecyltrialkoxysilan aufgebracht werden. Methoden zur Modifizierung von Oberflächen mit Aluminiumoxid werden unter anderem in US6,291,166 beschrieben.

Das erfindungsgemäße Verfahren zur Analyse von Cytosin-Methylierungsmustern besteht aus den folgenden Teilschritten:
1. Die genomische DNA wird aus Zellen oder anderen begleitenden Materialien isoliert und im wesentlichen irreversibel an eine Oberfläche gebunden.
2. Die an die Oberfläche gebundene DNA wird, bevorzugt mit einem Bisulfit (=Disulfit, Hydrogensulfit) derart behandelt, dass Cytosin in eine vom Basenpaarungsverhalten in der DNA-Duplex her unterschiedliche Base umgewandelt wird, während 5-Methylcytosin unverändert bleibt.
3. die im zweiten Schritt verwendeten Reagenzien werden in einem Waschschritt entfernt.
4. Ausgewählte Abschnitte der immobilisierten DNA werden in einer Polymerasereaktion amplifiziert und
5. Die Amplifikate werden hinsichtlich ihrer Sequenz untersucht.

In einer besonders bevorzugten Variante des Verfahrens werden zusätzlich die folgenden Schritte ausgeführt:
6. Die Reagenzien und Produkte der Polymerasereaktion werden in einem Waschschritt entfernt.
7. Weitere ausgewählte Abschnitte, welche von denen in Schritt d) verschieden sind, der immobilisierten DNA werden in einer Polymerasereaktion amplifiziert,
8. Die Amplifikate werden hinsichtlich ihrer Sequenz untersucht.

In einer weiteren besonders bevorzugten Variante des Verfahrens werden die Schritte 6-8 mehrmals wiederholt.

Im ersten Verfahrensschritt wird die bevorzugt genomische DNA aus Zellen oder anderen begleitenden Materialien isoliert und im wesentlichen irreversibel an eine Oberfläche gebunden.

Eine irreversible Bindung in Sinne der vorliegenden Erfindung meint eine Bindung, die mit den üblicherweise zur Verfügung stehenden Mitteln unter den in der Reaktion vorhandenen Bedingungen nicht wieder vollständig gelöst werden kann. Diese Bindung kann bevorzugt eine kovalente Bindung, eine Ionenpaarbindung oder aber eine Bindung sein, die auf elektrostatischen oder hydrophoben Effekten beruht.

Die Isolierung der DNA erfolgt bevorzugt derart, dass eine Körperflüssigkeit oder aber ein Lysat eines Gewebes mit der Oberfläche kontaktiert wird, die wiederum bevorzugt die DNA irreversibel bindet. Dazu ist im Falle des C18 Materials ein spezieller Puffer erforderlich (beispielsweise Triethylammoniumacetat). Der Überstand wird entfernt und es wird entweder mit Puffer oder Wasser (oder beidem) nachgewaschen, um die nachfolgende Bisulfit Reaktion mit so reinem Ausgangsmaterial wird möglich durchzuführen.

In einer besonders bevorzugten Variante des Verfahrens ist die Bindung der DNA an die Oberfläche kovalent. In einer weiteren besonders bevorzugten Verfahrensvariante wird die DNA unmittelbar in dem Immobilisierungsschritt auch isoliert. Die Isolierung der DNA erfolgt bevorzugt aus Vollblut oder Serum.

In einer weiteren besonders bevorzugten Verfahrensvariante erfolgt die Isolierung der DNA aus lysiertem Gewebe. Die Lysis wird besonders bevorzugt mittels Proteinase K durchgeführt.

In einer weiteren besonders bevorzugten Verfahrensvariante wird die Immobilisierung in den Gefäßen einer Mikrotiterplatte mit 96 Gefäßen oder 384 Gefäßen durchgeführt, wobei in den Gefäßen unterschiedliche DNA-Proben immobilisiert werden.

In einer besonders bevorzugten Verfahrensvariante wird die Immobilisierung in PCR-Reaktionsgefäßen durchgeführt, wobei in den Gefäßen unterschiedliche DNA-Proben immobilisiert werden.

Besonders bevorzugt erfolgt die Immobilisierung der DNA an einem Metalloxid, bevorzugt Aluminiumoxid. In einer weiteren besonders bevorzugten Verfahrensvariante erfolgt die Immobilisierung an einem hydrophoben Material und die Bindung ist nur unter den gewählten Pufferbedingungen im wesentlichen irreversibel.

Die zu analysierende DNA wird bevorzugt aus den üblichen Quellen für DNA erhalten, wie z. B. Zelllinien, Blut, Sputum, Stuhl, Urin, Gehirn-Rückenmarks-Flüssigkeit, in Paraffin eingebettetes Gewebe, beispielsweise Gewebe von Augen, Darm, Niere, Hirn, Herz, Prostata, Lunge, Brust oder Leber, histologische Objektträger und alle möglichen Kombinationen hiervon.

Im zweiten Schritt des Verfahrens behandelt man die an die Oberfläche gebundene DNA bevorzugt mit Bisulfit, (= Disulfit, Hydrogensulfit) derart, dass alle nicht an der 5-Position der Base methylierten Cytosine so verändert werden, dass eine hinsichtlich dem Basenpaarungsverhalten unterschiedliche Base entsteht, während die in 5-Position methylierten Cytosine unverändert bleiben.

Wird ein Bisulfitreagenz verwendet, so ist im Falle der C18 Oberfläche Trialkylammoniumbisulfit besonders bevorzugt, um eine feste Anbindung der DNA an die Oberfläche zu gewährleisten. Im Falle der Aluminiumoxidoberfläche wird bevorzugt Natriumbisulfit verwendet.

Die DNA-Probe denaturiert man besonders bevorzugt vor der Behandlung thermisch oder unter Verwendung eines alkalischen Reagenzes wie beispielsweise verdünnter (bevorzugt 0.1 bis 0.3 molarer) Natronlauge.

Wird für die Reaktion Bisulfit verwendet, so findet an den nicht methylierten Cytosinbasen eine Addition statt. Für das erfindungsgemäße Verfahren sind zudem bevorzugt ein denaturierendes Reagenz oder Lösungsmittel sowie ein Radikalfänger zugegen.

Dabei kommen als denaturierende Reagenzien oder Lösungsmittel bevorzugt die folgenden Verbindungen oder Verbindungsklassen in Frage:
Polyethylenglykoldialkylether, Dioxan und substituierte Derivate, Harnstoff oder Derivate, Acetonitril, primäre Alkohole, sekundäre Alkohole, tertiäre Alkohole, Diethylenglykoldialkylether, Triethylenglykoldialkylether, Tetraethylenglykol-dialkylether, Pentaethylenglykoldiakylether, Hexaethylenglykoldialkylether, DMSO oder THF. Allerdings ist auch die Einbettung der DNA in Agarose nach der Denaturierung möglich durch Zugabe derselben in gelöster Form und anschliessendes Abkühlen, analog zu der von Olek et al veröffentlichten Methode. Die Agarose kann nach der Bisulfitbehandlung mit heissem Puffer oder Wasser thermisch entfernt.

Die anschließende alkalische Hydrolyse (bevorzugt: Tris Puffer pH 10 oder Ammoniak) führt dann zur Umwandlung von nicht methylierten Cytosin-Nukleobasen in Uracil. Bevorzugt wird anschliessend die Desulfonierung der DNA (10-30 min, 90-100 °C) bei alkalischem pH-Wert durchgeführt.

Im dritten Schritt des Verfahrens werden die zuvor verwendeten Reagenzien in einem Waschschritt entfernt. Dabei ist es wiederum entscheidend, dass die immobilisierte, nunmehr chemisch behandelt vorliegende DNA an die Oberfläche gebunden bleibt. Dies ist einfach im Falle einer beispielsweise kovalenten Bindung an die Oberfläche. Dagegen ist wiederum ein entsprechender Puffer erforderlich, wenn eine Bindung über beispielsweise Triethylammoniumkationen an eine C18-Phase erfolgt. Dies erfordert dann auch in den Waschschritten einen Puffer, der die Bindung der DNA an die hydrophobe Phase fördert, wie beispielsweise ein Triethylammoniumacetat-Puffer.

Bevorzugt werden mehrere Waschschritte ausgeführt, die besonders bevorzugt aus einem automatisierten Pipettierschritt bestehen, in welchem Wasser oder Puffer zugegeben wird, und aus einem nachfolgenden Pipettierschritt, in dem der jeweilige Puffer oder das Wasser wieder entfernt wird. Dies kommt beispielsweise bei einer Immobilisierung der DNA in einer Mikrotiterplatte und bei Verwendung eines handelsüblichen Pipettierroboters (z. B. von den Firmen Tecan oder Qiagen) zum tragen.

Im vierten Verfahrensschritt werden ausgewählte Abschnitte der immobilisierten, behandelten DNA amplifiziert.

Man amplifiziert die DNA-Probe in einer Polymerasekettenreaktion, bevorzugt mit einer hitzebeständigen DNA-Polymerase. Die Amplifikation von mehreren DNA-Abschnitten wird vorzugsweise in einem Reaktionsgefäß gemacht.

Den Verfahrensschritt kann man auch vorzugsweise in zwei Teilschritten durchführen. Man beginnt mit einer PCR Präamplifikation mit mindestens einem Primerpaar unterschiedlicher Sequenz, die die vorbehandelte DNA Probe unspezifisch hybridisieren und daher im PCR Schritt mehr als ein Amplifkat ergeben. Danach führt man eine PCR Amplifikation des in der Präamplifikation gebildeten Produkts mit Primern unterschiedlicher Sequenz durch, die jeweils zu einem Abschnitt der vorbehandelten DNA-Probe [(+)-Strang oder (-)-Strang] identisch oder revers komplementär sind und die zu amplifizierende DNA spezifisch hybridisieren.

In einer besonders bevorzugten Verfahrensvariante wird ein Amplifikationsschritt mit mehreren Primerpaaren als Multiplex-PCR ausgeführt. Besonders bevorzugt ist es auch, dass alle oder ein großer Teil der Amplifikate einer immobilisierten DNA Probe gepoolt werden und so gemeinsam einer weiteren Analyse zugeführt werden. Besonders bevorzugt wird die Reaktionsmischung nach der Amplifikation aus dem Reaktionsgefäß, an welches die immobilisierte DNA gebunden ist, entfernt. Damit steht die immobilisierte DNA als Templat für weitere Amplifikationen, bevorzugt wiederum mit zuvor nicht verwendeten Primern, zur Verfügung.

In einer besonders bevorzugten Verfahrensvariante wird daher die Amplifikation mehrmals mit unterschiedlichen Primern wiederholt, so dass das Verfahren die folgenden zusätzlichen Schritte aufweist:
1) die Reagenzien und Produkte der Polymerasereaktion werden in einem Waschschritt entfernt.
2) weitere ausgewählte Abschnitte, welche von den zuvor amplifizierten verschieden sind, der immobilisierten DNA werden in einer Polymerasereaktion amplifiziert,
3) die Amplifikate werden hinsichtlich ihrer Sequenz untersucht.

In einer besonders bevorzugten Variante des Verfahren werden diese Schritte mehrmals wiederholt, wobei in jeder Amplifikation nach Schritt 2) andere Abschnitte amplifiziert werden als in einer der vorangegangenen Amplifikationen.

Im letzten Verfahrensschritt und auch wenn die obigen zusätzlichen Schritte ausgeführt werden, werden die Amplifikate jeweils hinsichtlich ihrer Sequenz untersucht. Dadurch kann unmittelbar auf den Methylierungsstatus ausgewählter Cytosinbasen in der DNA-Probe geschlossen werden.

Diese Sequenzanalyse und die nachfolgenden Schlussfolgerungen hinsichtlich des Methylierungsstatus können prinzipiell unter Verwendung vieler, dem Fachmann bekannter Methoden erfolgen, welche auch im Stand der Technik beschrieben sind.

Besonders bevorzugt erfolgt die Analyse durch Hybridisierung der Amplifikate an einen Oligonukleotidarray oder PNA (Peptide Nucleic Acids)-Array.

Es ist weiterhin bevorzugt, dass die Analyse während der Amplifikation durch eine Realtime PCR Methode erfolgt. Besonders bevorzugt ist damit eine Erfindungsvariante, in der von der DNA Extraktion, der Bisulfitbehandlung, der Amplifikation und der Detektion bevorzugt durch Realtime-PCR alle Schritte in einem Reaktionsgefäss ausgeführt werden können. Besonders bevorzugt in diesem Zusammenhang ist auch ein Verfahren, bei dem man die Analyse nach der Amplifikation im gleichen Reaktionsgefäß durch die Aufnahme einer Schmelzkurve durchführt und aus dem Schmelzverhalten auf die Basenzusammensetzung des Fragmentes und damit auf den Methylierungsstatus schließt.

Die Analyse kann auch durch Einbringen der Oberfläche in ein Massenspektrometer erfolgen, welches die Molekülmasse der Amplifikate, von Fragmenten der Amplifikate oder aber von Sonden, die spezifisch an die Amplifikate hybridisieren, bestimmt. Diese Information kann wiederum zur Identifizierung von Sequenzen herangezogen werden, wenn die Sequenz zum grossen Teil bereits bekannt ist. Es ist auch möglich, die gelösten Amplifikate separat in ein Massenspektrometer einzuführen und die Analyse nach dem Fachmann bekannten Methoden durchzuführen.

Besonders bevorzugt ist auch ein Verfahren, bei dem die Analyse durch allelspezifische Hybridisierung von Oligonukleotiden oder PNAs (Peptide Nucleic Acids) an die zu untersuchenden Positionen in den Amplifikaten erfolgt.

In einer weiteren besonders bevorzugten Variante des Verfahrens wird die Analyse durch Hybridisierung von Oligonukleotidprimern und einer nachfolgenden Primerextensionsreaktion oder einer Sequenzierreaktion durchgeführt.

Gegenstand der vorliegenden Erfindung ist auch die Verwendung des oben beschriebenen Verfahrens zur Diagnose und/oder Prognose nachteiliger Ereignisse für Patienten oder Individuen, wobei diese nachteiligen Ereignisse mindestens einer der folgenden Kategorien angehören: unerwünschte Arzneimittelwirkungen; Krebserkrankungen; CNS-Fehlfunktionen, Schäden oder Krankheit; Aggressionssymptome oder Verhaltensstörungen; klinische, psychologische und soziale Konsequenzen von Gehirnschädigungen; psychotische Störungen und Persönlichkeitsstörungen; Demenz und/oder assoziierte Syndrome; kardiovaskuläre Krankheit, Fehlfunktion und Schädigung; Fehlfunktion, Schädigung oder Krankheit des gastrointestinalen Traktes; Fehlfunktion, Schädigung oder Krankheit des Atmungssystems; Verletzung, Entzündung, Infektion, Immunität und/oder Rekonvaleszenz; Fehlfunktion, Schädigung oder Krankheit des Körpers als Abweichung im Entwicklungsprozess; Fehlfunktion, Schädigung oder Krankheit der Haut, der Muskeln, des Bindegewebes oder der Knochen; endokrine und metabolische Fehlfunktion, Schädigung oder Krankheit; Kopfschmerzen oder sexuelle Fehlfunktion.

Ebenso bevorzugt ist die Verwendung eines Verfahrens zur Unterscheidung von Zelltypen oder Geweben oder zur Untersuchung der Zelldifferenzierung.

Gegenstand der vorliegenden Erfindung ist auch ein Kit, bestehend aus einem Reagenz zur Behandlung der DNA, mindestens zwei Primeroligonukleotiden zur Herstellung der Amplifikate, einer Festphase zur Immobilisierung der Proben-DNA, sowie optional weiteren Lösungen und einer Anleitung zur Durchführung mindestens einer der oben beschriebenen Verfahrensvarianten.

### Beispiel:

### Bisulfit-Behandlung von Promega- und M13-DNA in derivatisierten Reaktionsgefäßen

### ANBINDUNG DER DNA

Für die Anbindung von DNA auf die mit Aluminiumoxid beschichtete Oberfläche der Reaktionsgefäße wurde EcoR1 geschnittene genomische DNA (Promega) und M13 Plasmid-DNA verwendet. Jeweils 160ng wurden in die entsprechenden Reaktionsgefäße pipettiert, mit Wasser auf ein Gesamtvolumen von 20µl aufgefüllt, auf einem Shaker kurz gemischt und für 15 Minuten bei Raumtemperatur inkubiert. Anschließend wurde die Lösung entfernt und die Gefäße zweimal mit je 50µl Wasser gewaschen. Um die Aktivität der verbleibenden Bindungsstellen auf der Tubeoberfläche herabzusetzen, wurden 10µl einer 5%igen Bovin-Serum-Albumin Lösung hinzu pipettiert, mit 40µl Wasser aufgefüllt und ebenfalls bei Raumtemperatur für 15 Minuten inkubiert. Abschließend wurden die Gefäße einmal mit jeweils 50µl Wasser gewaschen.

### BISULFIT-BEHANDLUNG

Die angebundene DNA wird bei 96°C ohne Zugabe von Wasser für 20 Minuten in einem Eppendorf-Mastercycler denaturiert. Die Gefäße werden alsdann schnellstmöglich entfernt und mit 6µl Dioxan versetzt, wodurch die Denaturierung der DNA erhalten bleibt. Für die Bisulfitreaktion wurden 10µl einer 0.75M Natriumbisulfit-Lösung, 2µl Radikalfänger (6-Hydroxy-2,5,7,8-tetramethylchroman-2-carbonsäure, 98,6mg in 1ml Dioxan) und 2µl Wasser zugegeben. Die Reaktionsgefäße wurden bei 50°C in einem Eppendorf-Mastercycler für fünf Stunden inkubiert.

### DESULFONIERUNG

Nach erfolgter Bisulfitreaktion wurden die Lösungen herauspipettiert und die Gefäße mit 100µl Wasser und, vorbereitend auf die Desulfonierung, mit 100µl einer 50mM Tris-HCl-Lösung gewaschen. Die Desulfonierung erfolgt mit 50µl einer 50mM Tris-HCl-Lösung bei pH9 für 20 Minuten bei 96°C. Nach dreimaligem Waschen mit je 50µl Wasser sind die Reaktionsgefäße einsatzbereit für eine Amplifikation mittels PCR.

### PCR

Die PCR wurde in einem Maßstab von 25µl angesetzt. Als Primer dienten für die Promega DNA 5'-TAA GTA TGT TGA AGA AAG ATT ATT GTA G-3' und 5'TAA AAA CTA TCC CAT AAT AAC TCC CAA C-3', sowie 5'-ATT ACA AAA TCG CGC AAA-3' und 5'-AAG TCG GAG GTT AAA AAG GT-3' (MWG) für die M13 Plasmid-DNA. Die beiden jeweiligen Primer wurden in einer Lösung mit einer Konzentration von 12.5pmol/µl vorgelegt, 2µl dieser Primerpaarlösung in das entsprechende Tube pipettiert. Für die PCR wurden 2.5µl dNTP-Mix (Fermentas, Konzentration je dNTP 2.5µmol/pl), 0.3µl Hot Star Taq (Qiagen), 2.5µl 10x PCR Buffer Solution (Qiagen, 15mMol MgCl₂ im Puffer enthalten) und 17.7µl Wasser (Fluka) pro Ansatz in die Gefäße gegeben.

Die Kontrolle der PCR erfolgte mittels Gelelektrophorese. Dazu wurden 5µl Probe mit 3µl Loading Dye auf ein 1.4% Agarosegel (Eurogentec., Inc.)aufgetragen, als Laufpuffer diente 1xTBE. Die Fragmente wurden mittels Ethidiumbromid angefärbt, das Gel im UV abfotografiert.

## Patentansprüche

1. Verfahren zur Analyse von Cytosin-Methylierungsmustern in genomischen DNA-Proben, wobei man die folgenden Verfahrensschritte ausführt:
a) die genomische DNA wird aus Zellen oder anderen begleitenden Materialien isoliert und im wesentlichen irreversibel an eine Oberfläche gebunden,
b) die an die Oberfläche gebundene DNA wird, bevorzugt mit einem Bisulfit (=Disulfit, Hydrogensulfit) derart behandelt, dass Cytosin in eine vom Basenpaarungsverhalten in der DNA-Duplex her unterschiedliche Base umgewandelt wird, während 5-Methylcytosin unverändert bleibt,
c) die in Schritt b) verwendeten Reagenzien werden in einem Waschschritt entfernt,
d) ausgewählte Abschnitte der immobilisierten DNA werden in einer Polymerasereaktion amplifiziert,
e) die Amplifikate werden hinsichtlich ihrer Sequenz untersucht.

2. Verfahren nach Anspruch 1, **dadurch gekennzeichnet, dass** man folgende zusätzliche Schritte ausführt:
f) die Reagenzien und Produkte der Polymerasereaktion werden in einem Waschschritt entfernt,
g) weitere ausgewählte Abschnitte, welche von denen in Schritt d) verschieden sind, der immobilisierten DNA werden in einer Polymerasereaktion amplifiziert,
h) die Amplifikate werden hinsichtlich ihrer Sequenz untersucht.

3. Verfahren nach Anspruch 2, **dadurch gekennzeichnet, dass** man die Schritte f)-g) mehrmals wiederholt, wobei in jeder Amplifikation nach Schritt g) andere Abschnitte amplifiziert werden als in einer der vorangegangenen Amplifikationen.

4. Verfahren nach einem der voranstehenden Ansprüche, **dadurch gekennzeichnet, dass** die Bindung der DNA an die Oberfläche kovalent ist.

5. Verfahren nach einem der voranstehenden Ansprüche, **dadurch gekennzeichnet, dass** man die DNA unmittelbar in dem Immobilisierungsschritt auch isoliert.

6. Verfahren nach einem der voranstehenden Ansprüche, **dadurch gekennzeichnet, dass** die Immobilisierung in PCR-Reaktionsgefäßen erfolgt, wobei in den Gefäßen unterschiedliche DNA-Proben immobilisiert werden.

7. Verfahren nach einem der voranstehenden Ansprüche, **dadurch gekennzeichnet, dass** die Immobilisierung der DNA an einem Metalloxid, bevorzugt Aluminiumoxid, erfolgt.

8. Verfahren nach einem der Ansprüche 1 bis 7, **dadurch gekennzeichnet, dass** die Immobilisierung an einem hydrophoben Material erfolgt und die Bindung nur unter den gewählten Pufferbedingungen im wesentlichen irreversibel ist.

9. Verfahren nach einem der voranstehenden Ansprüche, **dadurch gekennzeichnet, dass** ein Amplifikationsschritt mit mehreren Primerpaaren als Multiplex-PCR ausgeführt wird.

10. Verfahren nach einem der voranstehenden Ansprüche, **dadurch gekennzeichnet, dass** alle oder ein großer Teil der Amplifikate einer immobilisierten DNA Probe gepoolt werden und so gemeinsam einer weiteren Analyse zugeführt werden.

11. Verfahren nach Anspruch 9 oder 10, **dadurch gekennzeichnet, dass** es sich bei dieser weiteren Analyse um die Hybridisierung an einen Oligonukleotidarray oder PNA (Peptide Nucleic Acids)-Array handelt.

12. Verfahren nach einem der Ansprüche 1 bis 10, **dadurch gekennzeichnet, dass** die Analyse während der Amplifikation durch eine Realtime PCR Methode erfolgt.

13. Verfahren nach einem der Ansprüche 1 bis 10, **dadurch gekennzeichnet, dass** die Analyse nach der Amplifikation im gleichen Reaktionsgefäß durch die Aufnahme einer Schmelzkurve erfolgt.

14. Verfahren nach einem der Ansprüche 1 bis 10, **dadurch gekennzeichnet, dass** die Analyse durch allelspezifische Hybridisierung von Oligonukleotiden oder PNAs (Peptide Nucleic Acids) an die zu untersuchenden Positionen in den Amplifikaten erfolgt.

15. Verwendung eines Verfahrens nach einem der voranstehenden Ansprüche zur Unterscheidung von Zelltypen oder Geweben oder zur Untersuchung der Zelldifferenzierung.


Sequenzprotokoll

<110> Epigenomics AG

<120> Verfahren zum Nachweis von Cytosin-Methylierungen in immobilisierten
DNA Proben

<160> 4

<210> 1
<211> 28
<212> DNA
<213> Homo Sapiens

<400> 1

taagtatgtt gaagaaagat tattgtag                    28

<210> 2
<211> 28
<212> DNA
<213> Homo Sapiens

<400> 2

taaaaactat cccataataa ctcccaac                    28

<210> 3
<211> 18
<212> DNA
<213> Artificial Sequence

<220>
<223> Plasmid DNA

<400> 3

attacaaaat cgcgcaaa                    18

<210> 4

<211> 20
<212> DNA
<213> Artificial Sequence

<220>
<223> Plasmid DNA

<400> 4

aagtcggagg ttaaaaaggt                                                20